# EUROPÄISCHE PATENTSCHRIFT

(11) **EP 2 093 198 B1**
(45) Veröffentlichungstag und Bekanntmachung des Hinweises auf die Patenterteilung: **13.04.2011**
(21) Anmeldenummer: 09159020.8
(22) Anmeldetag: 25.09.2006
(51) Int. Cl.: C02F 9/02, C02F 103/36

(54) **Verfahren zur Behandlung von Abwasser aus Aldolisierungsverfahren**
Method for treating waste water from aldolisation processes
Procédé de traitement d'eaux usées à partir des procédés d'aldolisation

(30) Priorität: 30.09.2005 DE 102005047460
(43) Veröffentlichungstag der Anmeldung: 26.08.2009
(62) Teilanmeldung aus: 06793779.7
(73) Patentinhaber: BASF SE, 67056 Ludwigshafen (DE)
(72) Erfinder: Maaßen, Siegmar, 67065 Ludwigshafen (DE); Krokoszinski, Roland, 67273 Weisenheim a.Berg (DE); Kratz, Detlef, Singapore 268416 (SG)

(56) Entgegenhaltungen:
- EP-A- 1 496 043
- EP-A1- 0 631 988
- WO-A-03/070639
- GB-A- 960 936
- GB-A- 1 309 555

## Beschreibung

Die vorliegende Erfindung betrifft ein Verfahren zur Behandlung von mit wasserlöslichen organischen Verunreinigungen belastetem Abwasser aus einem Aldolisierungsverfahren mittels ein- oder mehrstufiger Extraktion des auf einen pH-Wert von 0 bis 6 eingestellten Aldolisierungsprozeßabwassers mit einer organischen Flüssigkeit.

Basenkatalysierte Aldolisierungsreaktionen, beispielsweise Aldoladditionen und Aldolkondensationen, haben bei der industriellen Herstellung von Alkoholen eine erhebliche Bedeutung erlangt. Lediglich beispielhaft sei die Herstellung von Neopentylglykol genannt, bei der im ersten Syntheseschritt Formaldehyd an Isobutyraldehyd unter Bildung von 2,2-Dimethyl-3-Hydroxy-propanal addiert wird. In noch weit größerem Maßstab werden die Weichmacheralkohole 2-Ethylhexanol bzw. 2-Propylheptanol hergestellt, bei deren ersten Synthesestufe zwei Moleküle n-Butyraldehyd bzw. n-Valeraldehyd in einer Aldolkondensation unter einhergehender Abspaltung von Wasser zu den ungesättigten Aldehyden 2-Ethylhexenal bzw. 2-Propylheptenal kondensiert werden, deren nachfolgende Hydrierung die betreffenden Weichmacheralkohole liefert (vgl. Weissermel, Arpe; Industrielle Organische Chemie; 4. Auflage, S. 150-152, 231-232, Wiley-VCH, Weinheim 1994).

Bei der basenkatalysierten Aldoladdition von Aldehyden zu β-Hydroxy-Aldehydaddukten bzw. der Aldolkondensation zweier Aldehyde zu den betreffenden α,β-ungesättigten Aldehyden, auch als Enalisierung bezeichnet, oder der Aldolkondensation von Aldehyden mit Ketonen, die entweder mittels einer wässrigen Base, z.B. Natriumhydroxid, katalysiert werden oder bei der Aufarbeitung einen Hydrofyseschritt oder eine Wasserextraktion zur Entfernung der Base aus dem Product benötigen, fallen beträchtliche Mengen an Abwasser an, das stark mit wasserlöslichen und in Wasser dispergierten, organischen Verbindungen belastet ist. Zwar sind diese organischen Verunreinigungen im Abwasser im Allgemeinen biologisch abbaubar, zum biologischen Abbau dieser Verunreinigungen, z.B. in einer Kläranlage, besteht aber ein relativ hoher Sauerstoffbedarf. Zur Abschätzung der Größe dieses Sauerstoffbedarfs wird der leicht zu ermittelnde CSB-Wert (CSB= Chemischer Sauerstoff-Bedarf) verwendet, zu dessen Bestimmung eine Wasserprobe mit einem Überschuss an Dichromat-Lösung oxidiert und das überschüssige Dichromat zurücktitriert wird (vgl. Deutsche Einheitsverfahren zur Wasser-, Abwasser- und Schlammuntersuchung, DIN 38406, Teil 44, Ausgabe Mai 1992). Infolge des zum Abbau eines Aldolisierungsprozeßabwassers in einer Kläranlage erforderlichen hohen Sauerstoffbedarfs muss folglich für einen entsprechend hohen Sauerstoffeintrag in das Abwasser gesorgt werden, was wiederum einen hohen Energieverbrauch für den erhöhten Eintrag von Luft ins Abwasser, gegebenenfalls verbunden mit einer Erweiterung der Kapazität der Kläranlage, erfordert. Ist dies nicht möglich, besteht die Gefahr, dass der CSB-Wert des aus der Kläranlage in den Vorfluter gelangenden Abwassers die gesetzlichen Vorgaben überschreitet oder der aerobe Abbau der im Abwasser enthaltenen organischen Stoffe mangels Sauerstoff zusammenbricht und eine unerwünschte anaerobe Zersetzung des Abwassers beginnt, was gemeinhin als Umkippen der Kläranlage bezeichnet wird.

Zur Vermeidung solch nachteiliger Folgen und der mit der Klärung eines solchen Abwassers verbundenen hohen Kosten wird hochbelastetes Abwasser aus Aldolisierungsanlagen zweckmäßigerweise einer Vorbehandlung zwecks Verringerung des CSB-Wertes unterzogen.

Eine solche Vorbehandlung eines Aldolisierungsprozeßabwassers wird beispielhaft für das bei der Aldolkondensation von n-Butyraldehyd zu 2-Ethylhexenal entstehende Abwasser in EP-A 631 988 beschrieben. Dabei wird das Aldolisierungsprozeßabwasser, gegebenenfalls nach Mischung mit anderen Waschwässern aus der 2-Ethylhexanol-Produktion auf einen pH-Wert von 0 bis 6 angesäuert, die sich dabei bildende organische Phase abgetrennt und die wässrige Phase mit einem C₈- bis C₁₆-Monoalkohol, im konkreten Fall mit 2-Ethythexanol, extrahiert. Das so behandelte Abwasser wird der Kläranlage zugeführt, während der die abgetrennten organischen Verunreinigungen - im wesentlichen Nebenprodukte aus der 2-Ethylhexanol-Produktion - enthaltende organische Extrakt zur Rückgewinnung des Extraktionsmittels - im vorliegenden Fall 2-Ethylhexanol - destilliert wird.

Nachteil der in EP-A 631 988 beschriebene Methode ist, dass bei der destillativen Wiedergewinnung des Extraktionsmittels die im Sumpf aufkonzentrierten, hochsiedenden Bestandteile dessen Rückgewinnung stören und zu einer stetigen Temperaturerhöhung im Sumpf der Destillationskolonne führen. Hohe Temperaturen wiederum verursachen die weitere Kondensation der hochsiedenden Verbindungen und schließlich Ablagerungen in der Destillationskolonne. Um die Sumpftemperaturen nicht über ein bestimmtes Maß ansteigen zu lassen, muss deshalb ein Teil des 2-Ethylhexanols mit im Sumpf der Kolonne verbleiben. Dieses 2-Ethylhexanol geht dann mit den hochsiedenden Bestandteilen bei der Entsorgung der Rückstände verloren, so dass dem in die Extraktion rezirkulierten 2-Ethylhexanol stets frisches 2-Ethylhexanol zugeführt werden muss.

Zur Vermeidung der geschilderten Nachteile des Verfahrens gemäß EP-A 631 988 schlägt EP-A 926 100 ein Verfahren vor, bei dem die verschiedenen Abwasserströme aus der 2-Ethylhexenal- und 2-Ethylhexanol-Produktion entweder einzeln oder miteinander vereinigt auf einen pH-Wert von 0 bis 6 eingestellt und anschließend zur Abtrennung der organischen Phase durch ein Koaleszierfilter geleitet werden. Die dabei erhaltene, von der Hauptmenge der organischen Verunreinigungen befreite wässrige Phase wird anschließend zur Abtrennung noch darin gelöster oder dispergierter organischer Bestandteile mit Hilfe eines C₈- bis C₁₆-Monoalkohols, im konkreten Fall 2-Ethylhexanol, extrahiert. Nach EP-A 926 100 soll durch diese Verfahrensweise der Anteil der hochsiedenden Verunreinigungen, die in den organischen Extrakt gelangen, so weit verringert werden, dass bei der anschließenden destillativen Rückgewinnung des Extraktionsmittels, z.B. 2-Ethylhexanol, die Verluste an Extraktionsmittel im Sumpf der Kolonne reduziert werden. Über das Ausmaß der Verluste an Extraktionsmittel enthält EP-A 926 100 keine Angaben, doch auch bei dem Verfahren gemäß EP-A 926 100 können Verluste an 2-Ethylhexanol nicht vermieden werden.

Der vorliegenden Erfindung lag somit die Aufgabe zugrunde ein verbessertes Verfahren zur Behandlung von mit wasserlöslichen organischen Verbindungen belastetem Abwasser aus einem Aldolisierungsverfahren zu finden, das es auf wirtschaftliche Weise und ökoeffizient ermöglicht, den chemischen Sauerstoffbedarf des Aldolisierungsprozeßabwassers so weit zu erniedrigen, dass es in einer Kläranlage problemlos abgebaut werden kann. Insbesondere, sollte der Einsatz eines teueren Extraktionsmittels und der verfahrenstechnische Aufwand zur möglichst verlustfreien, destillativen Rückgewinnung des Extraktionsmittels vermieden werden. Außerdem sollte das Verfahren für den kontinuierlichen getrieb geeignet sein.

Dementsprechend wurde ein Verfahren zur Behandlung von mit wasserlöslichen und/oder dispergierten organischen Verunreinigungen belastetem Abwasser aus einem Aldolisierungsverfahren mittels ein- öder mehrstufiger Extraktion des auf einen pH-Wert von 0 bis 6 eingestellten Aldolisierungsprozeßabwassers mit einer organischen Flüssigkeit gefunden, das dadurch gekennzeichnet ist, dass man als organische Flüssigkeit den organischen Leichtsiederaustrag aus der Destillation des nach Hydrierung von n-Butyraldehyd erhaltenen rohen n-Butanols, den organischen Leichtsiederaustrag aus der Destillation des nach Hydrierung von Isotiutyraldehyd erhaltenen rohen Isobutanols, den organischen Leichtsiederaustrag aus der Destillation des nach Hydrierung von n-Valeraldehyd erhaltenen rohen n-Pentanols, den organischen Leichtsiederaustrag aus der Destillation der nach Hydrierung von n-Valeraldehydllsovaleraldehyd-Gemischen erhaltenen rohen n-/iso-Pentanolgemische, den organischen Leichtsiederaustrag aus der Destillation des nach Hydrierung von 2-Ethylhexenal erhaltenen rohen 2-Ethylhexanols und/oder den organischen Leichtsiederaustrag aus der Destillation des nach Hydrierung von 2-Propylheptenal erhaltenen rohen 2-Propylheptanols zur Extraktion organischer Verunreinigungen aus dem angesäuerten Aldolisierungsprozessabwassers_einsetzt, die leichtflüchtigen organischen Verunreinigungen in einer Strippapparatur aus dem angesäuerten und extrahierten Aldolisierungsprozeßabwasser strippt und der Strippapparatur ein Abwasser entnimmt, das eine geringere Belastung an organischen Verunreinigungen hat als das der Extraktion zugeführte Aldolisierungsprozessabwasser.

Das erfindungsgemäß Verfahren unterscheidet sich fundamental von den Verfahren gemäß EP-A 631 988 und EP-A 926 100, indem
- als Extraktionsmittel kein Wertprodukt, wie z.B. 2-Ethylhexanol, eingesetzt wird, sondern der leichtsiedende, organische Vorlauf aus z.B. der Destillation von 2-Ethylhexanol, n-Butanol, Isobutanol, Pentanol und/oder 2-Propylheptanol, der ohnehin üblicherweise verbrannt wird;
- eine Rückgewinnung des Extraktionsmittels aus dem organischen Abwasserextrakt somit aus wirtschaftlichen Gründen nicht erforderlich ist;
- das extrahierte Abwasser von darin noch enthaltenem Extraktionsmittel leicht durch Strippung befreit und auf diese Weise dessen CSB-Wert weiter abgesenkt wird.

Eine energetisch aufwendige und mit Verlusten an Extraktionsmittel verbundene destillative Aufarbeitung des organischen Abwasserextrakts findet im erfindungsgemäßen Verfahren nicht statt.

Das erfindungsgemäß zu behandelnde Abwasser aus Aldolisierungsverfahren entsteht z. B. bei der Aldolkondensation von Aldehyden zu den entsprechenden α,β-ungesättigten Aldehyden, beispielsweise der Kondensation von n-Butyraldehyd zu 2-Ethylhexenal oder der Kondensation von n-Valeraldehyd zu 2-Propylheptenal, die mittels wässriger Alkalilauge katalysiert werden. Nach Verlassen des Kondensationsreaktors wird der wässrig-organische Reaktionsaustrag einer Phasenscheidung unterzogen, wobei sich eine organische, im wesentlichen aus dem gewünschten Aldolkondensationsprodukt bestehende Phase und eine wässrige Phase bildet. Während die organische Phase zu den gewünschten Endprodukten weiterverarbeitet wird, beispielsweise im Falle der Erzeugung von 2-Ethylhexenal durch Hydrierung zu 2-Ethylhexanol oder im Falle der Erzeugung von 2-Propylheptenal durch Hydrierung zu 2-Propylheptanol - muss die wässrige, mit organischen Verunreinigungen belastete Phase vor ihrer Einleitung in die Kläranlage in der Regel vorbehandelt werden. Je nach dem Ausmaß ihrer Wasserlöslichkeit können die in der wässrigen Phase befindlichen organischen Verunreinigungen vollständig oder zum Teil gelöst und/oder in Form fein verteilter, mikroskopisch kleiner Tröpfchen oder Partikel dispergiert vorliegen.

Als Nebenprodukte, die sich bei der Aldolkondensation bilden können und sich zum Teil gelöst oder dispergiert in der wässrigen Phase, zum anderen Teil gelöst in der das Aldolkondensationsprodukt enthaltenden, von der wässrigen Phase abgetrennten organischen Phase befinden können, seien beispielhaft und generisch die folgenden Verbindungsgruppen in der folgenden, nicht abschließenden Aufzählung aufgeführt:
- Isomere der bei der Aldolkondensation eingesetzten Carbonylverbindungen (Aldehyde, Ketone), insbesondere verzweigte Aldehyde;
- reduzierte Verbindungen der eingesetzten Carbonylverbindungen, insbesondere geradkettige und verzweigte Alkohole;
- Lactone, mit einer dem eingesetzten Aldehyd bzw. dem gebildeten Aldolkondensationsprodukt entsprechenden Anzahl an Kohlenstoffatomen;
- Alkohole, die ein Kohlenstoffatom mehr als die in die Aldolkondensation eingesetzte Carbonylverbindung haben sowie Alkohole mit der doppelten Anzahl an Kohlenstoffatomen als die eingesetzte Carbonylverbindung;
- Salze, insbesondere Alkalimetallsalze, von Carbonsäuren mit einer dem eingesetzten Aldehyd und/oder dem Aldolkondensationsprodukt entsprechenden Anzahl an Kohlenstoffatomen sowie durch die alkalische Verseifung der Lactone gebildete Salze der entsprechenden Hydroxycarbonsäuren;
- gesättigte Aldehyde mit der dem gebildeten Aldolkondensationsprodukt entsprechenden Anzahl an Kohlenstoffatomen;
- Carbonsäureester aus dem vorstehend genannten Carbonsäuren und Alkoholen;
- Acetale und Halbacetale der genannten Carbonylverbindungen mit den genannten Alkoholen.

Selbstverständlich können auch noch in die Aldolkondensation eingesetzte, jedoch nicht umgesetzte Carbonylverbindungen in der organischen und der wässrigen Phase des Aldolisierungsaustrags vorhanden sein.

Aufgrund der hohen Alkalinität der wässrigen Phase des Aldolisierungsaustrags liegen darin insbesondere die vorstehend genannten Salze in gelöster Form vor. Aufgrund ihrer zum Teil langen Alkylketten wirken diese Salze als Lösungsvermittler für andere schwerer wasserlösliche Nebenprodukte und erhöhen dadurch deren Gehalt in der wässrigen Phase, sei es in gelöster oder dispergierter Form.

Daher wird das Aldolisierungsprozeßabwasser vor seiner Behandlung nach dem erfindungsgemäßen Verfahren auf einen pH₋Wert von im Allgemeinen 0 bis 6, vorzugsweise von 1 bis 3, eingestellt. Diese Ansäuerung kann mit beliebigen starken Protonsäuren erfolgen, bevorzugt werden Mineralsäuren, wie Salzsäure, Schwefelsäure, Phosphorsäure oder Salpetersäure verwendet, besonders bevorzugt wird Schwefelsäure eingesetzt. Gewünschtenfalls können dem Abwasser aus dem Aldolisierungsverfahren vor dessen Ansäuerung noch mit organischen Stoffen belastete Abwässer aus anderen Prozessen zur weiteren Behandlung zugemischt werden, beispielsweise das nach der Kondensation des Kopfproduktes aus der n-Butanol-, Isobutanol-, Pentanol-, 2-Ethylhexanol und/oder 2-Propylheptanol-Destillation abgeschiedene Wasser. Bevorzugt wird jedoch allein das angesäuerte Aldolisierungsprozeßabwasser der Extraktion zugeführt. Die Ansäuerung des Aldolisienrngsprozeßabwassers kann in beliebigen zur Vermischung zweier Flüssigkeiten geeigneten Vorrichtungen erfolgen, beispielsweise in Rührkesseln oder statischen Mischern, bevorzugt werden statische Mischer eingesetzt. Der pH-Wert des Aldolisierungsprozeßabwassers kann z.B. mittels einer Glaselektrode gemessen werden.

Nach Ansäuerung des Aldolisierungsprozeßabwassers kann sich spontan eine organische Phase aus der wässrigen Phase separieren. Es ist möglich diese organische Phase vor Beginn der Extraktion abzutrennen, beispielsweise mittels eines Phasenscheiders, vorzugsweise wird aber das gesamte angesäuerte Aldolisierungsprozeßabwasser der Extraktion zugeführt.

Als Extraktionsmittel können im erfindungsgemäßen Verfahren vorteilhaft leichtsiedende, schwer wasserlösliche organische Abfallstoffe aus unterschiedlichen Produktionsprozessen verwendet werden. Die vorstehend erwähnten, als Extraktionsmittel im erfindungsgemäßen Verfahren geeigneten, leichtsiedenden, schwer wasserlöslichen organischen Abfallstoffe aus unterschiedlichen Produktionsverfahren sind der organische Leichtsiederaustrag aus der Destillation des nach Hydrierung von n-Butyraldehyd erhaltenen rohen n-Butanols, der organische Leichtsiederaustrag aus der Destillation des nach Hydrierung von Isobutyraldehyd erhaltenen rohen Isobutanols, der organische Leichtsiederaustrag aus der Destillation des nach Hydrierung von n-Valeraldehyd erhaltenen rohen n-Pentanols, der organische Leichtsiederaustrag aus der Destillation der nach Hydrierung von n-Valeraldehyd/Isovaleraldehyd-Gemischen erhaltenen rohen n-/iso-Pentanolgemische, der organische Leichtsiederaustrag aus der Destillation des nach Hydrierung von 2-Ethylhexenal erhaltenen rohen 2-Ethylhexanols oder der-organische Leichtsiederaustrag aus der Destillation des nach Hydrierung von 2-Propylheptenal erhaltenen rohen 2-Propylheptanols sein. Es können auch Gemische aus einzelnen oder allen der genannten organischen Leichtsiederausträge vorteilhaft als Extraktionsmittel im erfindungsgemäßen Verfahren benutzt werden. Zur Gewinnung dieser organischen Leichtsiederausträge kann so vorgegangen werden, dass man den betreffenden Hydrieraustrag, also das rohe Alkoholgemisch; dem unteren oder mittleren Teil einer Destillationskolonne zuführt, über den Kopf der Kolonne ein Leichtsiedergemisch abzieht, kondensiert und das zweiphasige, wässrige-organische Kondensat in einem Phasenscheider trennt, wobei die dabei erhaltene organische Phase erfindungsgemäß als organischer Leichtsiederaustrag zur Extraktion des angesäuerten Aldolisierungsprozeßabwassers verwendet werden kann, wohingegen der im wesentlichen von Leichtsiedern befreite Alkohol z.B. aus dem Sumpf oder über einen Seitenabzug der betreffenden Destillationskolonne entnommen werden kann. Obgleich die genannten Leichtsiedergemische aus technischer Sicht im Allgemeinen gleich gut zur Extraktion des angesäuerten Aldolisierungsprozeßabwassers geeignet sind, kann es im Einzelfall aufgrund einer besseren Verfügbarkeit am Standort der Aldolisierungsanlage vorteilhaft sein, im Falle der Aldolisierung von n-Birtyräldehyd zu 2-Ethylhexenal zur Extraktion des Aldolisierungsprozeßabwassers die genannten Leichtsiederausträge aus der n-Butanol-, Isobutanol- und/oder Ethylhexanol-Destillation einzusetzen, und im Falle der Aldolisierung von n-Valeraldehyd bzw. von Gemischen aus isomeren Valeraldehyden zu 2-Propylheptenal bzw. 2-Propylheptenal-Isomerengemischen die genannten Leichtsiederausträge aus der Destillation von n-Pentanol, Pentanolisomerengemischen und/oder 2-Propylheptanol einzusetzen.

Selbstverständlich können dem erfindungsgemäß einzusetzenden Extraktionsmittel auch noch andere Extraktionsmittel, wie C₅- bis C₁₀-Kohlenwasserstoffe, C₆- bis C₁₀-Alkohole, Carbonsäureester, z.B. Ethylacetat, Ether, z.B. Diethylether, Dipropylether, Diisopropylether, Dibutylether, Methyl-tert-butylether und/oder Ethyl-tert.-butylether, zugemischt werden, im Allgemeinen ist eine solche Zumischung aus technischen Gründen nicht erforderlich, weshalb die vorstehend genannten erfindungsgemäß einzusetzenden Extraktionsmittel bevorzugt ohne die Zumischung anderer Extraktionsmittel verwendet werden.

Entsprechend der Herkunft und Genese der erfindungsgemäß einzusetzenden Extraktionsmittel aus einem oder mehreren der vorstehend genannten Leichtsiederausträge - kann die Zusammensetzung der Extraktionsmittel hinsichtlich der darin enthaltenen Komponenten und deren Mengenanteilen ziemlich heterogen sein und kann im Zuge der kontinuierlichen Ausübung des Verfahrens stark variieren und zwar sowohl bezüglich des Gehalts an einzelnen Komponenten als auch bezüglich deren Mengenanteilen im Extraktionsmittel. Die nachstehenden Angaben zur Zusammensetzung des erfindungsgemäß einzusetzenden Extraktionsmittels dienen folglich allein der Veranschaulichung und Erläuterung des erfindungsgemäßen Verfahrens, und sind insbesondere nicht dazu gedacht, die Zusammensetzung des erfindungsgemäß einzusetzenden Extraktionsmittels festzulegen oder in irgendeiner Weise, sei es hinsichtlich der An- oder Abwesenheit einzelner Komponenten oder sei es hinsichtlich deren Mengenanteilen im Extraktionsmittel, zu beschränken. So kann das erfindungsgemäß einzusetzende Extraktionsmittel sämtliche der verschiedenen Butanol-Isomeren, weit überwiegend jedoch n-Butanol, Isobutanol und 2-Butanol, in beliebigen Konzentrationen, im Ällgemeinen in einem Anteil von 20 bis 50 Gew.-%, oder ein Gemisch isomerer Pentanole in beliebiger Konzentration, im Allgemeinen in einem Anteil von 20 bis 50 Gew.-%, Gemische aus C₅- bis C₁₁-Paraffinen, insbesondere Heptan, in Anteilen von 0 bis 30 Gew.-%, n- und iso-Butyraldehyd in Anteilen von 0 bis 5 Gew.-% und/oder n- und iso-Valeraldehyd in Anteilen von 0 bis 5 Gew.-%, 2-Ethylhexanol und/oder 2-Propylheptanol oder 2-Propylheptanol-Isomerengemisch in Anteilen von 0 bis 30 Gew.-% enthalten.

Die Extraktion des angesäuerten Aldolisierungsprozeßabwassers kann einstufig oder mehrstufig erfolgen.

Die einstufige Extraktion kann mit üblichen Extraktionsvorrichtungen durchgeführt werden, beispielsweise mit Mixer-Settler-Apparaturen in unterschiedlichen Ausgestaltungen. Als Mischorgan (Mixer) zur Vermischung des Extraktionsmittels mit dem angesäuerten Aldolisierungsprozeßabwasser kann hierbei ein statischer oder dynamischer Inline-Mischer, ein Rührapparat oder eine Pumpe dienen. Als Abscheider (Settler) können z. B. Schwerkraftabscheider mit oder ohne koaleszenzfördemden Einbauten verwendet werden, desgleichen Zentrifugalseparatoren oder Koaleszenzfilter als auch Kombinationen dieser Vorrichtungen. Es kann auch ein Zentrifugalextraktor vorteilhaft verwendet werden.

Bei der einstufigen Extraktion wird das angesäuerte Aldolisierungsprozeßabwasser im Mischorgan mit dem Extraktionsmittel vermischt. Das Extraktionsmittel kann dem Abwasser in Mengen von mindestens 0,001 kg pro Kilogramm Abwasser zugesetzt werden, im Allgemeinen wird das Extraktionsmittel in einer Menge von 0,01 bis 0,2 kg, vorzugsweise von 0,05 bis 0,1 kg pro Kilogramm Abwasser zugegeben. Selbstverständlich können dem Abwasser auch größere Mengen an Extraktionsmittel zugemischt werden. Die Extraktion kann bei Temperaturen von im Allgemeinen 5 bis 150°C, bevorzugt bei 25 bis 60°C und bei einem Druck von im Allgemeinen 0,1 bis 10 bar, vorzugsweise bei 0,5 bis 1,5 bar und besonders bevorzugt bei Atmosphärendruck durchgeführt werden.

Der so erhaltene organische Extrakt wird im erfindungsgemäßen Verfahren im Allgemeinen nicht mehr weiter aufgearbeitet, sondern z.B. zur Energiegewinnung verwertet, gewünschtenfalls kann der organische Extrakt aber auch hydriert und auf Wertprodukte aufgearbeitet werden. Bei einer solchen Hydrierung werden die im Extrakt enthaltenen Nebenprodukte aus der Aldolisierung im wesentlichen zu Alkoholen hydriert oder hydrogenolysiert. Hingegen wird die extrahierte Wasserphase zur weiteren Verringerung des Gehalts an noch darin enthaltenen organischen Stoffen, insbesondere von leichterflüchtigen organischen Stoffen, einer Strippung in einer Destillationsapparatur, vorzugsweise einer Destillationskolonne, unterzogen. Zur Strippung von leichterflüchtigen Komponenten wird das extrahierte Abwasser in den oberen Teil der Strippkolonne eingeleitet und dem nach unten fließenden Wasserstrom ein gasförmiges Strippmittel entgegengeführt, das in den unteren Teil der Kolonne eingeführt oder dort erzeugt wird. Bei dem Strippmittel kann es sich um ein unter den in der Strippkolonne herrschenden Bedingungen inertes Gas handeln, z.B. Stickstoff, vorzugsweise wird im erfindungsgemäßen Verfahren Wasserdampf als Strippmittel verwendet. Die Strippung kann im Allgemeinen bei Temperaturen von 50 bis 150°C und bei einem Druck von 0,1 bis 4 bar, vorzugsweise bei 0,5 bis 1,5 bar, durchgeführt werden. Die den Kopf der Strippkolonne verlassenden Brüden werden kondensiert und das Kondensat in einem Phasenscheider in eine organische und eine wässrige Phase geschieden, wohingegen nicht kondensierbare Gase z.B. über eine Fackel entsorgt werden können. Die so abgeschiedene Wasserphase kann vorteilhaft wieder in den oberen Teil der Strippkolonne zurückgeführt werden. Die so abgeschiedene organische Phase kann zur Energiegewinnung verwendet oder nach Hydrierung zur Gewinnung von Wertprodukten aufgearbeitet werden. Das so gereinigte Aldolisierungsprozeßabwasser kann dem Sumpf der Strippkolonne entnommen und nach Abkühlung in die Kläranlage gegeben werden. Die Wärme des heißen, aus dem Sumpf der Strippkolonne abgezogenen, gereinigten Aldolisierungsprozeßabwassers kann zur Aufheizung des der Strippkolonne aus der Extraktion zugeführten, extrahierten Aldolisierungsprozeßabwassers in einem Wärmetauscher benutzt werden.

Gewünschtenfalls können der Strippkolonne zusätzlich zum extrahierte Aldolisierungsprozeßabwasser auch andere mit flüchtigen organischen Bestandteilen belastete Abwässer zwecks Strippung zugeführt werden. Dabei kann es sich zum Beispiel um Abwässer aus der Butanol-, Isobutanol-, Pentanol-, 2-Ethylhexanol- und/oder 2-Propylheptanol-Destillation handeln, wie sie bei der Destillation der genannten hydrierten Produkte nach Kondensation und Phasenscheidung des Leichtsiederaustrags anfallen. Hierdurch wird es ermöglicht, die Belastung der Kläranlage mit organischen Stoffen ohne großen Aufwand zusätzlich abzusenken.

Von der Rückführung der nach Kondensation und Phasenscheidung der Brüden aus der Strippkolonne erhaltenen organischen Flüssigkeit als Extraktionsmittel in die Extraktionsstufe kann abgesehen werden, falls jederzeit relativ leichtflüchtige Extraktionsmittel aus anderen Prozessen, wie die vorstehend erwähnten organischen Leichtsiederausträge, günstig und in ausreichenden Mengen zur Verfügung stehen. In einem solchen Fall kann die so erhaltene organische Flüssigkeit beispielsweise zur Energiegewinnung verwertet werden.

Dementsprechend betrifft die vorliegende Erfindung ein Verfahren zur Behandlung von mit wasserlöslichen und/oder dispergierten organischen Verunreinigungen belastetem Abwasser aus einem Aldolisierungsverfahren mittels ein- oder mehrstufiger Extraktion des auf einen pH-Wert von 0 bis 6 eingestellten Aldolisierungsabwassers mit einer organischen Flüssigkeit, in dem man als organische Flüssigkeit den organischen Leichtsiederaustrag aus der Destillation des nach Hydrierung von n-Butyraldehyd erhaltenen rohen n-Butanols, den organischen Leichtsiederaustrag aus der Destillation des nach Hydrierung von Isobutyraldehyd erhaltenen rohen Isobutanols, den organischen Leichtsiederaustrag aus der Destillation des nach Hydrierung von n-Valeraldehyd erhaltenen rohen n-Pentanols, den organischen Leichtsiederaustrag aus der Destillation der nach Hydrierung von n-Valeraldehydilsovaleraldehyd-Gemischen erhaltenen rohen n-/iso-Pentanolgemische, den organischen Leichtsiederaustrag aus der Destillation des nach Hydrierung von 2-Ethylhexenal erhaltenen rohen 2-Ethylhexanols und/oder den organischen Leichtsiederaustrag aus der Destillation des nach Hydrierung von 2-Propylheptenal erhaltenen rohen 2-Propylheptanols zur Extraktion organischer Verunreinigungen aus dem angesäuerten Aldolisierungsprozessäbwassers einsetzt, die leichtflüchtigen organischen Verunreinigungen in einer Strippapparatur aus dem angesäuerten und extrahierten Aldolisierungsprozeßabwasser strippt und der Strippapparatur ein Abwasser entnimmt, das eine geringere Belastung an organischen Verunreinigungen hat als das der Extraktion zugeführte Aldolisierungsprozessabwasser.

Insbesondere falls ein sehr niedriger CSB-Wert des der Kläranlage zuzuführenden Aldolisierungsprozeßabwassers gefordert ist, kann es vorteilhaft sein, die Extraktion des angesäuerten Aldolisierungsprozeßabwassers in einer mehrstufigen, beispielsweise einer zwei-, drei- oder vierstufigen Extraktion, durchzuführen, wobei das Extraktionsmittel besonders vorteilhaft im Gegenstrom zum zu extrahierenden Abwasser geführt werden kann.

Für die mehrstufige Extraktion des angesäuerten Aldolisierungsprozeßatiwassers können die Extraktionsapparaturen, wie sie für die einstufige Extraktion geschildert wurden, ebenfalls verwendet werden. Hierzu werden zwei oder mehr der genannten Extraktionsapparäte in Reihe geschaltet. Eine Mehrstufigkeit der Extraktion kann aber auch durch die Verwendung einer Gegenstrom-Extraktionskolonne im erfindungsgemäßen Verfahren erzielt werden. Eine bevorzugte Ausführungsform der mehrstufigen Extraktion bei der Ausübung des erfindungsgemäßen Verfahrens ist die Verwendung einer zweistufigen Mixer-Settler-Kaskade mit Gegenstromführung der wässrigen und organischen Phase. Die Extraktionsbedingungen entsprechen hinsichtlich Druck, Temperatur und verwendbarem Extraktionsmittel denjenigen wie sie für die einstufige Extraktion angegeben wurden.

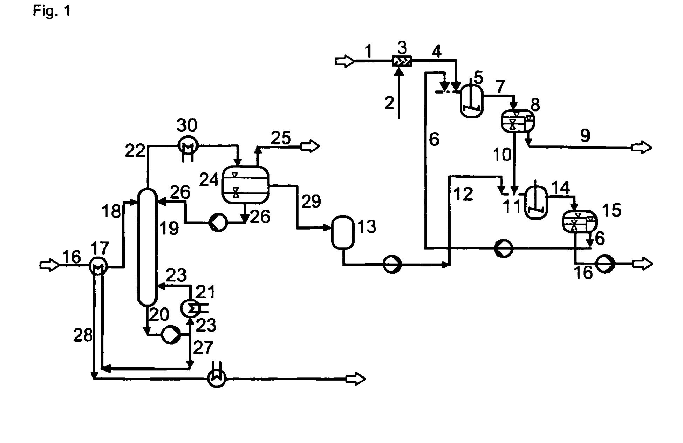

Anhand der schematischen Zeichnung Fig. 1, wird im Folgenden beispielhaft das Verfahrensprinzip einer vorteilhaften Ausgestaltung des erfindungsgemäßen Verfahrens veranschaulicht, ohne dass dieser beispielhaften Darstellung ein einschränkender Charakter bezüglich der Anwendung und/oder Ausgestaltung des erfindungsgemäßen Verfahrens zukommt.

Das Aldolisierungsprozeßabwasser gelangt über Leitung 1 in den statischen Mischer 3, wo es mit einer über Leitung 2 zugeführten Mineralsäure, beispielsweise Schwefelsäure, vermischt und auf den gewünschten pH-Wert eingestellt wird. Das angesäuerte Aldolisierungsprozeßabwasser wird über Leitung 4 in das Mischorgan 5, in der vorliegenden Zeichnung ein Rührkessel, geleitet, der über Leitung 6 mit Extraktionsmittel aus der zweiten Extraktionsstufe versorgt wird. Das im Mischorgan 5 erhaltene Aldolisierungsprozeßabwasser-/Extraktionsmittel-Gemisch wird über Leitung 7 in den Phasenscheider 8, gemäß Zeichnung ein Schwerkraftphasenscheider, überführt und scheidet sich dort in eine wässrige und eine organische Phase. Die organische Phase wird über Leitung 9 beispielsweise zur Energiegewinnung entsorgt, während die wässrige Phase über Leitung 10 in die zweite Extraktionsstufe, bestehend aus dem Mixer (Rührkessel) 11 und dem über Leitung 14 damit verbundenen Settler (Schwerkraftphasenscheider) 15, eingeführt wird. Das Mischorgan 11 erhält fisches Extraktionsmittel aus dem Sammelbehälter 13 über Leitung 12. Das im Phasenscheider abgetrennte Extraktionsmittel wird im Gegenstrom über Leitung 6 in die erste Extraktionsstufe mit dem Mixer 5/Settler 8 geführt, wohingegen das angesäuerte und extrahierte Aldolisierungsprozeßabwasser über die Leitungen 16 und 18, nach Vorerwärmung im Wärmetauscher 17, in den oberen Teil der Strippkolonne 19 gepumpt wird. Die Strippkolonne 19 wird mittels des Umlaufverdampfers 21 beheizt und zu diesem Zweck ein Aldolisierungsprozeßabwasserstrom über Leitung 20, den Verdampfer 21 und Leitung 23 in die Kolonne zirkuliert. Der durch den Verdampfer 21 erzeugte Wasserdampf strippt die leichtflüchtigen, organischen Verunreinigungen aus dem in Kolonne 19 befindlichen Aldolisierungsprozeßabwasser, die aus dem Kopf der Kolonne 19 über Leitung 22 ausgetragen werden. Die Wasserdampf und gestrippte organische Komponenten enthaltenden Brüden werden im Kühler 30 kondensiert und im Phasenscheider 24 in eine Gasphase, eine organische Phase und eine Wasserphase getrennt. Die nichtkondensierbaren Bestandteile der Brüden werden über Leitung 25 beispielsweise zu einer Fackel oder einer anderen Verbrennungsvorrichtung entsorgt. Die Wasserphase wird über Leitung 26 in den oberen Teil der Strippkolonne 19 zurückgeführt und zusammen mit über Leitung 18 zugeführtem Aldolisierungsprozeßabwasser im Gegenstrom mit dem vom Verdampfer 21 erzeugten Wasserdampf gestrippt. Im stationären Zustand des Verfahrens, wird ein Teilstrom des aus dem Sumpf der Kolonne über Leitung 20 abgezogenen, gestrippten Aldolisierungsprozeßabwassers über Leitung 27 abgezweigt, im Wärmetauscher 17 zur Vorheizung des aus der Extraktion kommenden Aldolisierungsprozeßabwassers verwendet und über Leitung 28, gegebenenfalls nach weiterer Abkühlung, in die Kläranlage gegeben.

Die im Phasenscheider 24 abgetrennte organische Flüssigkeit gelangt in einer nichterfindungsgemäßen Variante des Verfahrens über Leitung 29 in den Sammelbehälter 13, der als Extraktionsmittelreservoir dient. Im Sammelbehälter 13 werden alle im erfindungsgemäßen Verfahren verwendeten Extraktionsmittel, beispielsweise die Leichtsiederausträge aus der n-Butanol-, Isobutanol- und/oder 2-Ethylhexanol-Destillation, zusammengeführt (nicht eingezeichnet).

Soll die Strippkolonne 19 auch zur Strippung von leichtflüchtige organische Bestandteile enthaltenden Abwässern aus anderen Verfahren, die keiner Ansäuerung bedürfen, benutzt werden, werden diese zweckmäßigerweise dem Aldolisierungsprozeßabwasserstrom in Leitung 16 zugemischt.

Die folgenden Beispiele dienen der Veranschaulichung der Erfindung.

### Beispiele

### Analytik:

Der gesamte organisch-gebundene Kohlenstoff im Abwasser ("TOC" = total organic carbon) wurde bei den nachfolgenden Beispielen nach EN 1484 - H3 aus dem Jahr 1997 bestimmt. Der chemische Sauerstoffbedarf ("CSB") wurde nach DIN 38409, Teil 44 (Methode H44 der Deutschen Einheitsverfahren zur Wasser-, Abwasser- und Schlammuntersuchung, Mai 1992) bestimmt.

### Beispiel 1:

1500 g Abwasser (Feed, F, pH 13,6) aus der Enalisierung zu 2-Ethylhexenal mit einem CSB-Wert von 26800 mg/l (TOC = 7820 mg/l) wurden mit 150 g konzentrierter Schwefelsäure auf pH 2 eingestellt und anschließend bei Atmosphärendruck und bei einer Temperatur von 25°C mit 41 g eines Leichtsiedergemisches (Solvens, S) bei einem Phasenverhältnis von S:F = 0,025 g/g intensiv durchmischt. Das Solvensgemisch hatte eine Zusammensetzung von je etwa 37 Ges.-% n-Butanol, 27 Gew.-% i-Butanol, 15 Gew.-% n-Heptan, 8 Gew.-% bzw. 1 Gew.-% i- und n-Butyraldehyd, 7,5 Gew.-% Wasser und 4,5 Gew.-% weitere organische Nebenkomponenten. Nach Trennung der Phasen wurde diese Extraktionsprozedur wiederholt. 1488 g des resultierenden extrahierten Abwassers wurden bei 1012 mbar einer Wasserdampfdestillation unterzogen bis die Siedetemperatur und die Kondensationstemperatur der entstandenen Brüden jeweils ca. 100°C erreichten. Dabei wurden 116 g zweiphasiges Destillat gewonnen. Die organische Phase (54 g) bestand aus 77 Gew.-% Butanolen, 18 Gew.-% Wasser sowie Buttersäure und Butyraldehyden, die wässrige Phase (62 g) aus 89 Gew.-% Wasser und 10 Gew.% Butanolen sowie wenigen Nebenkomponenten.
Das so entstandene, behandelte Abwasser hatte noch einen CSB-Wert von 3870 mg/l (TOC = 1300 mg/l).

### Beispiel 2:

Im Mischer (V = 0,001 m³) eines einstufigen, kontinuierlich betriebenen Mixer-Settlers wurden 24 kg/h Abwasser (Feed, F, pH 13,6) aus der Enalisierung zu 2-Ethylhexenal mit einem CSB-Wert von 19600 mg/l (TOC = 6900 mg/l) mit 1,34 kg/h konzentrierter Schwefelsäure auf pH 2 eingestellt und bei Atmosphärendruck und bei einer Temperatur von 40°C mit 1,94 kg/h des in Beispiel 1 genannten Leichtsiedergemisches (Solvens, S, Phasenverhältnis von S : F = 0,056 g/g) intensiv durchmischt. Die entstehende Dispersion wurde anschließend in einen Schwerkraftabscheider (V = 0,002 m³) kontinuierlich in zwei klare Phasen getrennt. 1160 g des resultierenden extrahierten Abwassers wurden bei ca. 1013 mbar einer Wasserdampfdestillation unterzogen bis die Siedetemperatur und die Kondensationstemperatur der entstehenden Brüden jeweils ca. 100°C erreichten.
Das so entstandene, behandelte Abwasser hatte noch einen CSB-Wert von 2500 mg/l (TOC = 800 mg/l).

## Patentansprüche

1. Verfahren zur Behandlung von mit wasserlöslichen und/oder dispergierten organischen Verunreinigungen belastetem Abwasser aus einem Aldolisierungsverfahren mittels ein- oder mehrstufiger Extraktion des auf einen pH-Wert von 0 bis 6 eingestellten Aldolisierungsprozeßabwassers mit einer organischen Flüssigkeit, **dadurch gekennzeichnet, dass** man als organische Flüssigkeit den organischen Leichtsiederaustrag aus der Destillation des nach Hydrierung von n-Butyraldehyd erhaltenen rohen n-Butanols, den organischen Leichtsiederaustrag aus der Destillation des nach Hydrierung von Isobutyraldehyd erhaltenen rohen Isobutanols, den organischen Leichtsiederaustrag aus der Destillation des nach Hydrierung von n-Valeraldehyd erhaltenen rohen n-Pentanols, den organischen Leichtsiederaustrag aus der Destillation der nach Hydrierung von n-Valeraldehydllsovaleraldehyd-Gemischen erhaltenen rohen n-/iso-Pentanolgemische, den organischen Leichtsiederaustrag aus der Destillation des nach Hydrierung von 2-Ethylhexenal erhaltenen rohen 2-Ethylhexanols und/oder den organischen Leichtsiederaustrag aus der Destillation des nach Hydrierung von 2-Propylheptenal erhaltenen rohen 2-Propylheptanols zur Extraktion organischer Verunreinigungen aus dem angesäuerten Aldolisierungsprozessabwassers einsetzt, die leichtflüchtigen organischen Verunreinigungen in einer Strippapparatur aus dem angesäuerten und extrahierten Aldolisierungsprozeßabwasser strippt und der Strippapparatur ein Abwasser entnimmt, das eine geringere Belastung an organischen Verunreinigungen hat als das der Extraktion zugeführte Aldolisierungsprozessabwasser.

## Claims

1. A process for the treatment of wastewater from an aldolization process which is contaminated with water-soluble and/or dispersed organic impurities by means of single-stage or multistage extraction with an organic liquid of the aldolization process wastewater which has been set to a pH of from 0 to 6, wherein the organic low boiler fraction from the distillation of the crude n-butanol obtained after hydrogenation of n-butyraldehyde, the organic low boiler fraction from the distillation of the crude isobutanol obtained after hydrogenation of isobutyraldehyde, the organic low boiler fraction from the distillation of the crude n-pentanol obtained after hydrogenation of n-valeraldehyde, the organic low boiler fraction from the distillation of the crude n-pentanol/isopentanol mixtures obtained after hydrogenation of n-valeraldehyde/isovaleraldehyde mixtures, the organic low boiler fraction from the distillation of the crude 2-ethylhexanol obtained after hydrogenation of 2-ethylhexenal and/or the organic low boiler fraction from the distillation of the crude 2-propylheptanol obtained after hydrogenation of 2-propylheptenal is used as organic liquid for the extraction of organic impurities from the acidified aldolization process wastewater, the volatile organic impurities are stripped from the acidified and extracted aldolization process acidified and extracted aldolization process wastewater in a stripping apparatus and a wastewater having a lower content of organic impurities than the aldolization process wastewater fed to the extraction is taken off from the stripping apparatus.

## Revendications

1. Procédé pour le traitement d'eaux usées polluées par des impuretés organiques solubles et/ou dispersées dans l'eau provenant d'un procédé d'aldolisation au moyen d'une extraction à une ou plusieurs étapes des eaux usées du procédé d'aldolisation réglées à un pH de 0 à 6 avec un liquide organique, **caractérisé en ce qu'**on utilise comme liquide organique le produit évacué à bas point d'ébullition, organique provenant de la distillation du n-butanol brut obtenu après l'hydrogénation de n-butyraldéhyde, le produit évacué à bas point d'ébullition, organique provenant de la distillation de l'isobutanol brut obtenu après l'hydrogénation d'isobutyraldéhyde, le produit évacué à bas point d'ébullition, organique provenant de la distillation du n-pentanol brut obtenu après l'hydrogénation de n-valéraldéhyde, le produit évacué à bas point d'ébullition, organique provenant de la distillation de mélanges de n-pentanol/isopentanol bruts obtenus après l'hydrogénation de mélanges de n-valéraldéhyde/isovaléraldéhyde, le produit évacué à bas point d'ébullition, organique provenant de la distillation du 2-éthylhexanol brut obtenu après l'hydrogénation de 2-éthylhexénal et/ou le produit évacué à bas point d'ébullition, organique provenant de la distillation du 2-propylheptanol brut obtenu après l'hydrogénation de 2-propylhepténal pour l'extraction d'impuretés organiques des eaux usées acidifiées du procédé d'aldolisation, on épuise les impuretés organiques à bas point d'ébullition dans un appareil d'épuisement des eaux usées, acidifiées et extraites du procédé d'aldolisation et on prélève de l'appareil d'épuisement des eaux usées qui présentent une plus faible pollution en impuretés organiques que les eaux usées du procédé d'aldolisation introduites dans l'extraction.
